(19) Europäisches Patentamt
European Patent Office
Office européen des brevets

(11) EP 4 671 297 A1

(12) # EUROPEAN PATENT APPLICATION

(43) Date of publication:
**31.12.2025 Bulletin 2026/01**

(21) Application number: **24184969.4**

(22) Date of filing: **27.06.2024**

(51) International Patent Classification (IPC):
***C08G 63/08*** *(2006.01)*

(52) Cooperative Patent Classification (CPC):
**C08G 63/08**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Johannes Gutenberg-Universität Mainz**
**55122 Mainz (DE)**

(72) Inventors:
- **Frey, Holger**
**55128 Mainz (DE)**
- **Matthes, Rebecca**
**55128 Mainz (DE)**
- **Dreier, Philip**
**55128 Mainz (DE)**

(74) Representative: **Durm Patentanwälte PartG mbB**
**Patentanwälte**
**Moltkestrasse 45**
**76133 Karlsruhe (DE)**

(54) **USE OF BLOCK COPOLYMERS COMPRISING A POLY(ETHYLENE OXIDE) WITH A C1 TO C3-ALKYLOXYMETHYL SIDE CHAINS BLOCK AND HYDROPHOBIC BLOCKS IN PARTICLES AND IN MEDICAL DEVICES**

(57) The present invention refers to the use of novel polyoxyalkylene based block copolymers in particles or in medical devices, wherein the particles comprise at least one novel polyoxyalkylene based block copolymer and preferably at least one active agent.

EP 4 671 297 A1

## Description

## Technical Field

The present invention refers to the use of polyoxyalkylene based block copolymers in particles or in medical devices. The particles comprising at least one novel polyoxyalkylene based block copolymer and preferably at least one active agent.

## Background

Amphiphilic block copolymers comprising hydrophilic polyethylene oxide) and hydrophobic polymer blocks are important materials in the medical device industry and pharmaceutical technology. Monomers that are typically used for the hydrophobic blocks are inter alia lactides, glycolide and caprolactone. The hydrophilic block is typically poly(ethylene oxide) (PEO). PEO is known for its biocompatibility and the so-called "stealth effect" that avoids recognition by the patient's reticuloendothelial system. Hydrophilicity of the block copolymers can be tailored by molecular weight of the hydrophilic and hydrophobic blocks and monomers chosen for the hydrophobic segments. The number and order of blocks in the polymer chain is also important for the properties. AB and ABA type block copolymers are typically used. Self-organization of the amphiphilic molecules enables self-organization in aqueous media to nano-scaled polymeric micelles. The assemblies (micelles) comprise a hydrophobic polyester core and a hydrophilic polyether corona. They are used as carriers for actives and dyes in pharmaceutical technology. Amphiphilic block copolymers are further used in medical device industry because of the improved hydrophilicity of the devices compared to using solely hydrophobic polymers. The improved hydrophilicity leads to faster uptake of water and degradation caused by hydrolysis. Fine control of hydrophilicity enables control over degradation speed and mechanical properties of the device.

A major concern of PEO is the immune response of the patient's body that can lead to the formation of anti-PEG antibodies (APA). APAs can cause adverse reactions, i.e. allergic reactions, and the loss of the "stealth effect" resulting in decrease of functionality of the drug. Copolymers of poly(ethylene oxide) and polyethylene oxide) having C1 to C3-alkyloxymethyl side chains are a class of polymers that can be tailored to exhibit similar physicochemical characteristics compared to PEO homopolymers.

Therefore, there is a need for novel amphiphilic block copolymers that do not show the formation of anti-PEO antibodies, i.e. immunogenic potential, which are suitable in the medical device industry and pharmaceutical technology.

The inventors of the present invention found that the block copolymers of the present invention can fulfill this need. The randomized polyethylene glycol) copolymer (rPEG) structure of the block copolymers of the present invention impedes the interaction with anti-PEG antibodies due to the increased spatial requirements of the alkyloxy methyl side chains. Combination of hydrophilic rPEG blocks with hydrophobic polymer blocks leads to amphiphilic block copolymers. The block copolymers show similar or improved functionality in medical device industry and pharmaceutical technology without the immunogenic potential. Moreover, in the novel block copolymers it is possible to control the number and type (C1 to C3) of alkoxy methyl side chains inside the rPEG blocks. This enables additional control over hydrophilicity and crystallinity of the materials compared to using solely PEO. In particular, the novel block copolymers show miscibility of the two polymer phases and absence of crystallinity. This can lead to desirable characteristics like matrix homogeneity, faster and homogeneous water uptake and hydrolyzation, or absence of phase mixing and phase transitions that can impact storage stability.

## Summary of the invention

Therefore, the present invention refers to the use of a block copolymer in a particle or a medical device, wherein the block copolymer is obtained or obtainable by reacting at least one monomer selected from L-lactide, D-lactide, DL-lactide, epsilon-caprolactone, trimethylene carbonate, delta-valerolactone, alpha-methylene-delta-valerolactone, aspartic acid, glutamic acid, glycolide, and para-dioxanone, or by reacting a prepolymer of the structure:

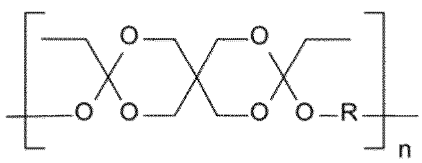
,

wherein R are independently of each other straight, branched or cyclic C1 to C20 alkylene groups; with a prepolymer H-[A]-$R^1$;

wherein [A] is a polyoxyalkylene group comprising at least one unit

(a)

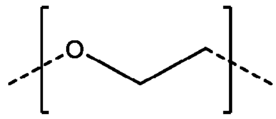

and at least one unit selected from the group of

(b)

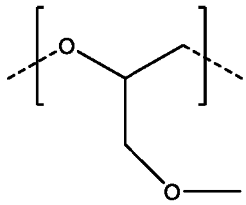,

(c)

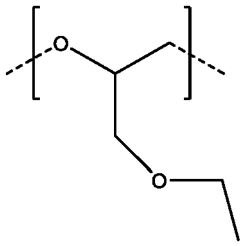,

(d)

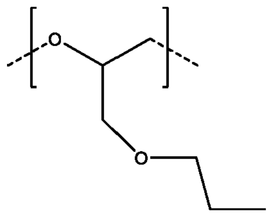,

and

(e)

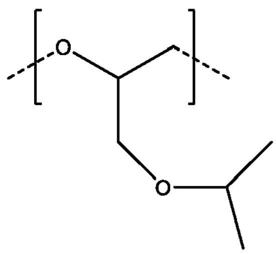;

wherein

$R^1$ is selected from -H, -OH, -SH, $-NH_2$, $-NHR^2$, $-NR^3R^4$, $-OR^5$, $-SR^5$ or linear, branched or cyclic alkyl groups having up to 20 carbon atoms; and wherein

$R^2$ to $R^5$ are independently selected from linear, branched or cyclic alkyl groups having up to 20 carbon atoms, in which up to 5 carbon atoms can be substituted with an oxygen or a sulfur atom; with the proviso that for the use in a particle at least one of the at least one monomer(s) is selected from epsilon-caprolactone, delta-valerolactone, alpha-methylene-delta-valerolactone, aspartic acid, glutamic acid, glycolide, and para-dioxanone, if [A] does not comprise unit (b).

These and other aspects, embodiments, features, and advantages of the invention will become apparent to a person skilled in the art through the study of the following detailed description and claims. Any feature from one aspect of the invention can be used in any other aspect of the invention. Furthermore, it will readily be understood that the examples contained herein are intended to describe and illustrate the invention but not to limit the invention and that, in particular, the invention is not limited to these examples.

**Detailed description of the invention**

As used herein, the following terms have the meanings ascribed to them unless specified otherwise.

Unless the context requires otherwise, throughout the present specification and claims, the word "comprise" and variations thereof, such as, "comprises", "comprising", "contain", and "containing" are to be construed in an open and inclusive sense, that is, as "including, but not limited to".

Unless defined otherwise, all technical and scientific terms used herein have the same meaning as is commonly understood by one of skill in the art. As used in the specification and claims, the singular form "a", "an" and "the" include plural references unless the context clearly dictates otherwise.

Numerical ranges that are indicated in the format "from x to y" also include the stated values. If several preferred numerical ranges are indicated in this format, it is self-evident that all ranges that result from the combination of the various endpoints are also included.

"One or more", as used herein, relates to at least one and comprises 1, 2, 3, 4, 5, 6, 7, 8, 9 or more of the referenced species. Similarly, "at least one" means one or more, i.e., 1, 2, 3, 4, 5, 6, 7, 8, 9 or more. "At least one", as used herein in relation to any component, refers to the number of chemically different molecules, i.e. to the number of different types of the referenced species, but not to the total number of molecules. For example, "at least one therapeutic agent" means that at least one type of molecule falling within the definition for a therapeutic agent is used but that also two or more different types of therapeutic agents falling within this definition can be present, but does not mean that only one or more molecules of one type of therapeutic agents are present.

All percentages given herein in relation to the compositions relate to wt.-% relative to the total weight of the respective composition, if not explicitly stated otherwise.

"Essentially free of" according to the present invention with regard to compounds means that the compound can only be present in an amount, which does not influence the characteristics of the composition, in particular the respective compound is present in less than 3 wt.-%, preferably 1 wt.-%, more preferably 0.01 wt.-%, based on the total weight of the composition or is not present at all.

The term "nucleic acid(s)" as used herein refers to a compound(s) containing at least two deoxyribonucleotides or ribonucleotides in either single- or double- or triple-stranded form and includes DNA, RNA, and hybrids thereof. DNA may be in the form of antisense molecules, plasmid DNA (pDNA), linear or circular DNA, PCR products, or vectors. RNA may be in the form of self-amplifying RNA (saRNA) or small hairpin RNA (shRNA), small interfering RNA (siRNA), chemically modified or unmodified messenger RNA (mRNA), antisense RNA, circular RNA (circRNA) comprising at least one coding sequence, micro RNA (miRNA), micRNA, multivalent RNA, transfer RNA (tRNA), single guided RNA (sgRNA), replicating RNA (repRNA), dicer substrate RNA or viral RNA (vRNA), antisense oligonucleotide (ASO), double-stranded RNA (dsRNA) and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (e.g., degenerate codon substitutions), alleles, orthologs, single nucleotide polymorphisms, and complementary sequences as well as the sequence explicitly indicated.

An "effective amount" or "therapeutically effective amount" of an active agent such as a nucleic acid is an amount sufficient to produce the desired effect, e.g., an increase or inhibition of expression of a target sequence in comparison to the normal expression level detected in the absence of the nucleic acid. An increase in expression of a target sequence is achieved when any measurable level is detected in the case of an expression product that is not present in the absence of the nucleic acid. In the case where the expression product is present at some level prior to contact with the nucleic acid, an in increase in expression is achieved when the fold increase in value obtained with a nucleic acid such as mRNA relative to

control is about 1.05, 1.1, 1.2, 1.3, 1.4, 1.5, 1.75, 2, 2.5, 3, 4, 5, 6, 7, 8, 9, 10, 15, 20, 25, 30, 40, 50, 75, 100, 250, 500, 750, 1000, 5000, 10000, or greater. Inhibition of expression of a target gene or target sequence is achieved when the value obtained with a nucleic acid such as antisense oligonucleotide relative to the control is about 95%, 90%, 85%, 80%, 75%, 70%, 65%, 60%, 55%, 50%, 45%, 40%, 35%, 30%, 25%, 20%, 15%, 10%, 5%, or 0%. Suitable assays for measuring expression of a target gene or target sequence include, e.g., examination of protein or RNA levels using techniques known to those of skill in the art such as dot blots, northern blots, in situ hybridization, ELISA, immunoprecipitation, enzyme function, fluorescence, or luminescence of suitable reporter proteins, as well as phenotypic assays known to those of skill in the art.

A "stereoisomer" refers to a compound made up of the same atoms bonded by the same bonds but having different three-dimensional structures, which are not interchangeable. The present invention contemplates various stereoisomers and mixtures thereof and includes "enantiomers", which refers to two stereoisomers whose molecules are non-superimposable mirror images of one another.

A "tautomer" refers to a proton shift from one atom of a molecule to another atom of the same molecule. The present disclosure includes tautomers of any said compounds.

"Pharmaceutically acceptable salt" includes both acid and base addition salts.

The block copolymer of the present invention is obtained or obtainable by reacting

at least one monomer, preferably one or two monomers, selected from L-lactide, D-lactide, DL-lactide, epsilon-caprolactone, trimethylene carbonate, delta-valerolactone, alpha-methylene-delta-valerolactone, aspartic acid, glutamic acid, glycolide, and para-dioxanone, or by reacting a prepolymer

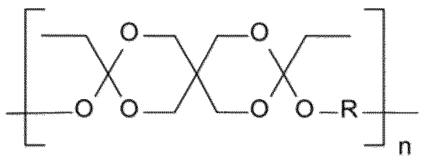
,

wherein R are independently of each other straight, branched or cyclic C1 to C20 alkylene groups; with a prepolymer H-[A]-$R^1$;

wherein [A] is a polyoxyalkylene group comprising at least one unit

(a)

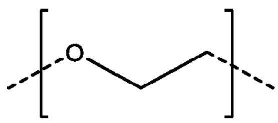

and at least one unit selected from the group of

(b)

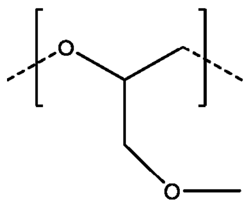
,

(c)

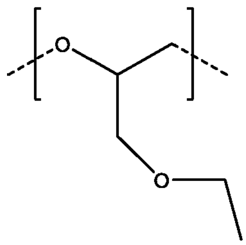

,

(d)

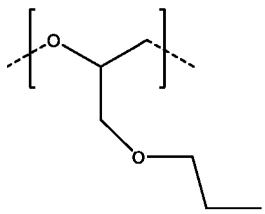

,

and

(e)

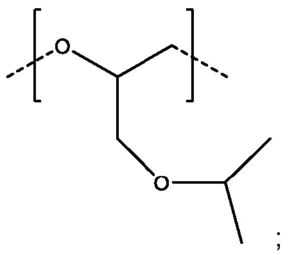

;

wherein

$R^1$ is selected from -H, -OH, -SH, $-NH_2$, $-NHR^2$, $-NR^3R^4$, $-OR^5$, $-SR^5$ or linear, branched or cyclic alkyl groups having up to 20 carbon atoms; and wherein

$R^2$ to $R^5$ are independently selected from linear, branched or cyclic alkyl groups having up to 20 carbon atoms, in which up to 5 carbon atoms can be substituted with an oxygen or a sulfur atom; preferably in the presence of a catalyst. Polyorthoester of the structure

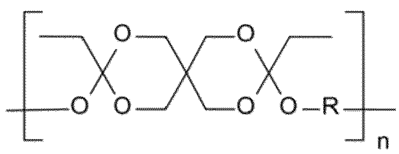

,

wherein R are independently of each other straight, branched or cyclic C1 to C20 alkylen groups are well known to the expert in the art and may be prepared by polymerization of 3,9-diethyliden-2,4,8,10-tetraoxaspiro(5.5)undecane with a diol. n is preferably 5 to 500; with the proviso that for the use in a particle at least one of the at least one monomer(s) is selected from epsilon-caprolactone, delta-valerolactone, alpha-methylene-delta-valerolactone, aspartic acid, glutamic acid, glycolide, and para-dioxanone, if [A] does not comprise unit (b).

In one embodiment the block copolymer has a number average molecular weight of 1,200 to 600,000 g/mol, preferably 2,000 to 250,000 g/mol, more preferably 5,000 to 100,000 g/mol, preferably determined via size exclusion chromatography using a refractive index (RI) detector and polyethylene oxide standards. The size-exclusion chromatography can preferably be performed with dimethylformamide (DMF with 1 g/L LiBr) as the mobile phase (flow rate 1 mL/min) on poly(2-hydroxyethylmethacrylat) (PHEMA) 300/100/40 columns at 50 °C, with polymer concentrations of 1 mg/mL. Calibration is carried out using polyethylene oxide standards (from Polymer Standard Service, Mainz, Germany).

In one embodiment the catalyst is a ring opening catalyst or a polycondensation catalyst, preferably the ring opening catalyst is a tin catalyst, more preferably tin (II) 2-ethylhexanoate, or preferably the polycondensation catalyst is an organic acid, more preferably p-toluenesulfonic acid.

Polycondensation reactions and ring opening polymerizations reactions are well known to the skilled person in the field. Suitable polycondensation reaction conditions are for example disclosed in WO 2022/152835 A1 and suitable ring opening polymerization conditions are for example disclosed by D. Bendix in Polymer Degradation and Stability, Volume 59, Issues 1-3, 3 January 1998, Pages 129-135.

In one embodiment the at least one monomer, preferably one or two monomers, is selected from L-lactide, D-lactide, DL-lactide, epsilon-caprolactone, trimethylene carbonate, delta-valerolactone, alpha-methylene-delta-valerolactone, glycolide, and para-dioxanone, preferably from L-lactide, D-lactide, DL-lactide, epsilon-caprolactone, and glycolide, more preferably from L-lactide, D-lactide, DL-lactide, and glycolide.

In one embodiment, when the at least one monomer is two monomers, one is selected from the group of L-lactide, D-lactide, DL-lactide, in the following referred to as lactide, and the other one is glycolide, the amount of lactide and glycolide can vary. In this embodiment, [A] comprises preferably at least one unit (b). In a further embodiment the amounts are 1 to 99 mole %, 40 to 99 mole %, 50 to 99 mole %, 60 to 99 mole %, 70 to 99 mole %, or 80 to 99 mole % lactide and from 1 to 99 mole %, 1 to 60 mole %, 1 to 50 mole %, 1 to 40 mole %, or 1 to 20 mole % of glycolide, wherein the amount of lactide and glycolide is 100 mole %, wherein all amounts in mole % are based on the sum of the moles of all lactides and glycolide. In a further embodiment the mole ratios of lactide to glycolide is: 95:5 (polylactide-co-glycolide), 85:15 poly(lactide-co-glycolide), 75:25 poly(lactide-co-glycolide), 65:35 poly(lactide-co-glycolide), or 50:50 poly(lactide-co-glycolide).

In one embodiment, when the at least one monomer is two monomers, one is selected from the group of L-lactide, D-lactide, DL-lactide, in the following referred to as lactide, and the other one is caprolactone, the amount of lactide and caprolactone can vary. In a further embodiment the mole ratios of lactide to caprolactone can be 95:5 poly(lactide-co-caprolactone), 85:15 poly(lactide-co-caprolactone), 75:25 poly(lactide-co-caprolactone), 65:35 poly(lactide-co-caprolactone), 50:50 poly(lactide-co-caprolactone), 40:60 poly(lactide-co-caprolactone), 25:75 poly(lactide-co-caprolactone), 10:90 poly(lactide-co-caprolactone), or 5:95 poly(lactide-co-caprolactone).

In one embodiment H-[A]-$R^1$ has a number average molecular weight of 200 to 200,000 g/mol, preferably 500 to 100,000 g/mol, more preferably 1,000 to 50,000 g/mol, preferably determined via size exclusion chromatography using a refractive index (RI) detector and polyethylene oxide standards. The size-exclusion chromatography can preferably be performed with dimethylformamide (DMF with 1 g/L LiBr) as the mobile phase (flow rate 1 mL/min) on poly(2-hydroxyethylmethacrylat) (PHEMA) 300/100/40 columns at 50 °C, with polymer concentrations of 1 mg/mL. Calibration is carried out using polyethylene oxide standards (from Polymer Standard Service, Mainz, Germany).

In one embodiment the polyoxyalkylene group [A] comprises or consist of at least one unit

(a)

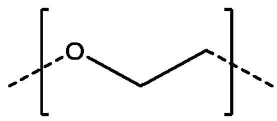

and at least one unit

(b)

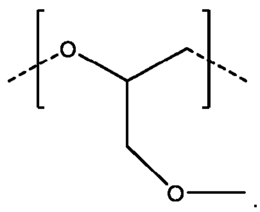

In a preferred embodiment, the amount of units (a) and (b) is at least 3 mole % each, more preferably at least 10 mole %, based on the overall number of moles of units (a) to (e) in [A]. In one preferred embodiment, the at least one monomer is not selected from the group consisting of L-lactide, D-lactide, and DL-lactide, if [A] does not comprise unit (b). In one embodiment, at least one of the at least one monomer(s) is selected from epsilon-caprolactone, delta-valerolactone, alpha-methylene-delta-valerolactone, aspartic acid, glutamic acid, glycolide, and para-dioxanone, if [A] does not comprise unit (b). In one embodiment, at least one of the at least one monomer(s) is selected from epsilon-caprolactone, delta-valerolactone, alpha-methylene-delta-valerolactone, aspartic acid, glutamic acid, and para-dioxanone, if [A] does not comprise unit (b). In one embodiment, the at least one monomer is two monomers and the two monomers are not lactide and glycolide, if [A] does not comprise unit (b). In a very preferred embodiment the use of the present invention has the proviso that at least one of the at least one monomer(s) is selected from epsilon-caprolactone, delta-valerolactone, alpha-methylene-delta-valerolactone, aspartic acid, glutamic acid, glycolide, and para-dioxanone, if [A] does not comprise unit (b).

In one embodiment $R^1$ is $-OR^5$, preferably wherein $R^5$ is selected from linear, branched or cyclic alkyl groups having up to 20 carbon atoms, in which up to 5 carbon atoms can be substituted with an oxygen atom.

The block copolymer of the present invention has the structure $[B]-[A]-R^1$;

wherein [A] is a polyoxyalkylene group comprising at least one unit

(a)

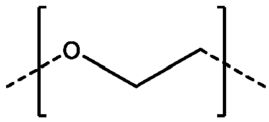

and at least one unit selected from the group of

(b)

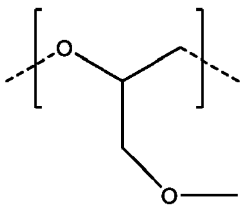
,

(c)

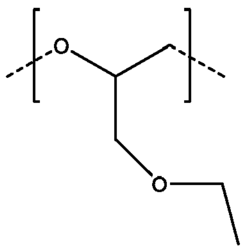
,

(d)

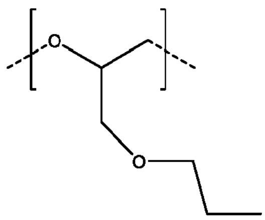
,

and

(e)

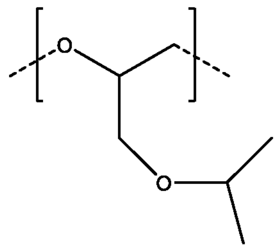

;

wherein

$R^1$ is selected from -H, -OH, -O-[B], -SH, $-NH_2$, $-NHR^2$, $-NR^3R^4$, $-OR^5$, $-SR^5$ or linear, branched or cyclic alkyl groups having up to 20 carbon atoms; and wherein

$R^2$ to $R^5$ are independently selected from linear, branched or cyclic alkyl groups having up to 20 carbon atoms, in which up to 5 carbon atoms can be substituted with an oxygen or a sulfur atom; and

the rest(s) [B] is (are independently of each other) a homopolymer or a copolymer comprising at least one monomer selected from the group consisting of L $[-CH(CH_3)C(=O)O-]$ (e.g. derived from L-lactide), D $[-CH(CH_3)C(=O)O-]$ (e.g. derived from D-lactide), DL $[-CH(CH_3)C(=O)O-]$ (e.g. derived from DL-lactide), $[-O-(CH_2)_5C(=O)-]$ (e.g. derived from epsilon-caprolactone), $[-O-(CH_2)_3-O-C(=O)-]$ (e.g. derived from trimethylene carbonate), $[-O-(CH_2)_4C(=O)-]$ (e.g. derived from delta-valerolactone), $[-O-(CH_2)_3-C(=C)-C(=O)-]$ (e.g. derived from alpha-methylene-delta-valerolactone), $[-CH(CH_2-COOH)-C(=O)-NH-]$ (e.g. derived from aspartic acid, $[-CH(COOH)-CH_2-CH_2-C(=O)-NH-]$ (e.g. derived from glutamic acid), $[-O-CH_2-C(=O)-]$ (e.g. derived from glycolide), and $[-O-CH_2-CH_2-O-CH_2-C(=O)-]$ (e.g. derived from para-dioxanone) or [B] is a prepolymer of the structure

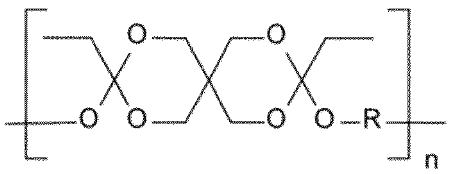

,

wherein R are independently of each other straight, branched or cyclic C1 to C20 alkylene groups (e.g. derived from polymerization of 3,9-diethyliden-2,4,8,10-tetraoxaspiro(5.5)undecane with a diol HO-R-OH); with the proviso that for the use in a particle at least one of the at least one monomer(s) is selected from epsilon-caprolactone, delta-valerolactone, alpha-methylene-delta-valerolactone, aspartic acid, glutamic acid, glycolide, and para-dioxanone, if [A] does not comprise unit (b). Preferably [B] comprises at least 95 % by weight, more preferably 98 % by weight and most preferably 100 % by weight of these monomers. In one embodiment [B] is a homopolymer.

The prepolymer $H-[A]-R^1$ is preferably obtained by an anionic ring opening copolymerisation of

ethylene oxide
with
at least one comonomer selected from
2-(methoxymethyl)oxirane (glycidyl methyl ether), 1,2-epoxy-3-ethoxypropane, 1,2-epoxy-3-n-propoxypropane, 1,2-epoxy-3-iso-propoxypropane, preferably the at least one comonomer is 2-(methoxymethyl)oxirane; and
an initiator, which is suitable to form $-R^1$ as defined above for the bock copolymer, the initiator is preferably 1-methoxy-3-(2-methoxyethoxy)propan-2-ol, in the presence of a base.

The base is preferably a base having a pka of at least 16, preferably of at least 19, more preferably is potassium tert-butoxide.

In a preferred embodiment, a small amount of pure ethylene oxide is added after the copolymerization step so that 2 to 5 additional units derived from ethylene oxide are present at one or both ends of A. If it is desired to use a prepolymer which has 2 to 5 additional units derived from ethylene oxide present at the end with $R^1$, the initiator may be chosen to be $^-O(CH_2CH_2O)_n-R^1$, with n = 2 to 5.

The synthesis of $HOCH_2CH_2-A-R^1$ via anionic ring-opening copolymerization is for example described in PCT/EP2022/062896, in the document $HOCH_2CH_2-A-R^1$ is referred to as polymer, which is incorporated by reference.

The anionic ring-opening copolymerization is preferably performed at a temperature in the range of -10 to 90 °C, more preferably -10 to 70 °C, most preferably -10 to 60°C.

The polyoxyalkylene group [A] comprises unit (a) and at least one of units (b) to (e). In one embodiment the polyoxyalkylene group [A] essentially consists or consists of unit (a) and at least one of units (b) to (e). In particular, the polyoxyalkylene group [A] is essentially free of residues, or is free of residues. Preferably, the polyoxyalkylene group [A] comprises at least 95% by weight, more preferably are least 98% by weight and most preferably 100% by weight of monomers (a) to (e).

In one preferred embodiment the polyoxyalkylene group [A] comprises or consists of units (a) and (b) and optionally further units selected from (c) to (e). In one preferred embodiment the polyoxyalkylene group [A] comprises or consists of units (a) and (b).

For the sake of clarity, the passage comprises unit (a) does not mean that only one unit (a) is present, but that at least one unit of (a) are present in the group, i.e., several monomeric units derived from ethylene oxide can be present. For example, 1 to 20 units (a) can be present in group [A]. The same applies to the passage at least one unit (b) to (e). However, it can be that only one (number) unit (a) or only one (number) unit (b) to (e) is present in group [A], if not explicitly defined otherwise.

In a preferred embodiment unit (a) makes up 5 to 95% of group [A], based on the number of all monomer units (a) to (e) of group [A], whereas the other units (b) to (e) add up to the remainder. In a further preferred embodiment unit (b) is present in up to 70% of group [A], more preferably unit (b) is present in 30 to 70% of group [A], most preferably unit (a) is additionally present in 30 to 70%, adding up to 100%.

In a preferred embodiment the molar ratio of (a) to the sum of moles of (b) to (e), is 1 to 9 to 9 to 1, preferably 2 to 8 to 8 to 2, more preferably 3 to 7 to 7 to 3. More preferably the molar ratio of (a) to (b), is 1 to 9 to 9 to 1, preferably 2 to 8 to 8 to 2, more preferably 3 to 7 to 7 to 3.

In a preferred embodiment the dispersity (PDI) of -[A]-$R^1$ is 1.15 or less, more preferably 1.10 or less, most preferably 1.08 or less, wherein preferably the weight average and the number average molecular weight are determined with size-exclusion chromatography as described for Mw above. The size-exclusion chromatography can preferably be performed with dimethylformamide (DMF with 1 g/L LiBr) as the mobile phase (flow rate 1 mL/min) on poly(2-hydroxyethylmethacrylat) (PHEMA) 300/100/40 columns at 50 °C. Polymer concentrations are 1 mg/mL. Calibration is carried out using polyethylene oxide standards (from Polymer Standard Service, Mainz, Germany).

The group $R^1$ can be modified by choosing a suitable initiator and/or by chemically modifying the end group initially formed during the anionic ring opening copolymerisation in the production of the prepolymer H-[A]-$R^1$. Such reactions are well known in the art. $R^1$ can for example be a functional group selected from acetal (dialkoxy), aldehyde (formyl), amide (carboxamido), azide, carbonate (alkoxycarbonyl)oxy), carboxyl (carboxy), carboxylic anhydride, ester (alkoxycarbonyl), ether, halo, haloformyl (carbonohaloridoyl), hemiacetal (alkoxyol), hemiketal (alkoxyol), hydroxy, imide (imido), imine (imino), ketal (dialkoxy), ketone (oyl), orthoester (trialkoxy), primary, secondary, tertiary amino group, primary, secondary and tertiary alkoxy group, sulfhydryl (sulfanyl, H-S-), thioether and combinations thereof. In a preferred embodiment the end group is selected from the group consisting of alkyl, hydrogen, hydroxy, alkoxy, sulfanyl, phthalimide, amide, amine and combinations thereof. The end group $R^1$ can be a primary alkoxy group selected of the formulae R-$(CH_2)_n$-O-, wherein R is linear, branched or cyclic alkyl, preferably with 1 to 20 carbon atoms or phenyl and n equals 1 to 20.

In a preferred embodiment $R^1$ is -$OR^5$, wherein $R^5$ is selected from linear, branched or cyclic alkyl groups having up to 20 carbon atoms, in which up to 5 carbon atoms can be substituted with an oxygen atom; more preferably $R^1$ is selected from methoxy, ethoxy, propoxy, butoxy, pentoxy, hexoxy, heptoxy, octoxy, nonoxy, decanoxy, 2-ethylhexoxy, dodecan-1-oxy, 1-methoxy-3-(2-methoxyethoxy)propan-2-oxy, 1-octadecanoxy, 3-methylbutan-1-oxy, phenylmethanoxy, 3-ethyl-butoxy, and 2,3-dialkoxypropoxy, 1-methoxy-3-(2-methoxyethoxy)propoxy. The end group can be introduced by a suitable initiator, which can be alkyl anions and hydride anions, like metal alkyl or metal hydride compounds of the above-mentioned end groups -$R^1$. Preferably however the alkoxy anion and the thioalkoxy anion are not tertiary alkoxy or thioalkoxy anions. The imide anion is preferably a phthalimide anion. The metal counterion is preferably $Na^+$, $K^+$ or $Cs^+$. In one embodiment the initiator is a salt of $MeOCH_2CH_2O^-$, $MeO(CH_2CH_2O)_2^-$, $BenzylOCH_2CH_2O^-$, $BzO(CH_2CH_2O)_2^-$, $(Bz)_2N-CH_2CH_2O^-$, $(Bz)_2N-(CH_2CH_2O)_2^-$, phthalimide-$CH_2CH_2O^-$, phthalimide-$(CH2CH2O)_2^-$, wherein Me is methyl and Bz is benzyl. Most preferred are $MeO(CH_2CH_2O)_2^-$, $BzOCH_2CH_2O^-$ and $(Bz)_2N-CH_2CH_2O^-$.The counter ion is preferably $Na^+$, $K^+$ or $Cs^+$. The initiator may be provided in an inert solvent. The solvent is preferably a non-protic solvent and most preferably dimethyl sulfoxide (DMSO) or toluene. Furthermore, the copolymerization reaction is preferably performed in the same solvent.

The end-group fidelity of the group -A-$R^1$ of the present invention may be determined on the respective prepolymer H-[A]-$R^1$ by MALDI TOF or by a combination of MALDI TOF with $^1H$ NMR by known methods. The group -[A]-$R^1$ of the present invention has preferably an end-group fidelity of at least 95 %, more preferably of at least 98 %.

The polyoxyalkylene group [A] of the present invention may be a random copolymer. Such groups provide the lowest immunogenicity, as they do not provide a blueprint for the immune system for antibodies. They are intrinsically resistant to an immune response and are therefore preferred embodiments of the present invention.

In an alternative embodiment the polyoxyalkylene group [A] of the present invention may have a block like

structure or a tapered or gradient structure. The methods to prepare such polymers are known to the skilled person in the field of polyoxyalkylenes. In such embodiments it is preferred that no more than 5% of group [A], based on the overall number of monomer units of group [A], comprise blocks with more than 15 ethylene oxide derived repeating units, more preferably that no more than 5% of the macromolecules of the polymers comprise blocks with more than 8 ethylene oxide derived repeating units.

Often crystallizations produce a solvate of a block copolymer used in the present invention. As used herein, the term "solvate" refers to an aggregate that comprises one or more molecules of a compound with one or more molecules of solvent. The solvent may be water, in which case the solvate may be a hydrate.

Alternatively, the solvent may be an organic solvent. Thus, the block copolymers used in the present invention may exist as a hydrate, including a monohydrate, dihydrate, hemihydrate, sesquihydrate, trihydrate, tetrahydrate and the like, as well as the corresponding solvated forms. Solvates of block copolymers used in the present invention may be true solvates, while in other cases the block copolymers used in the invention may merely retain adventitious water or be a mixture of water plus some adventitious solvent.

The block copolymer used in the present invention may be produced by a method comprising or consisting of the steps:

providing a prepolymer H-[A]-$R^1$;
wherein [A] is a polyoxyalkylene group comprising at least one unit

(a)

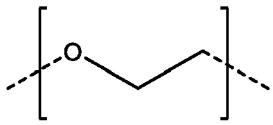

and at least one unit selected from the group of
(b)

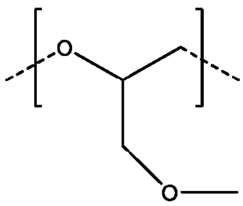

,

(c)

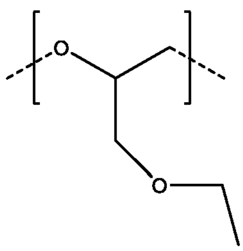

,

(d)

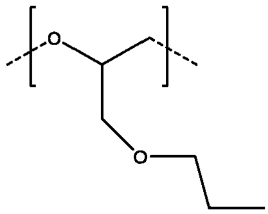,

and

(e)

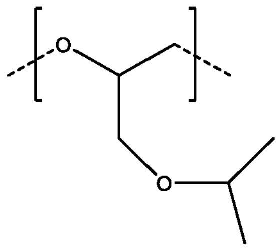;

wherein

$R^1$ is selected from -H, -OH, -SH, $-NH_2$, $-NHR^2$, $-NR^3R^4$, $-OR^5$, $-SR^5$ or linear, branched or cyclic alkyl groups having up to 20 carbon atoms; and wherein

$R^2$ to $R^5$ are independently selected from linear, branched or cyclic alkyl groups having up to 20 carbon atoms, in which up to 5 carbon atoms can be substituted with an oxygen or a sulfur atom; and allowing the prepolymer to react with at least one monomer, preferably one or two monomers, selected from L-lactide, D-lactide, DL-lactide, epsilon-caprolactone, trimethylene carbonate, delta-valerolactone, alpha-methylene-delta-valerolactone, 3,9-diethyliden-2,4,8,10-tetraoxaspiro(5.5)undecane, aspartic acid, glutamic acid, glycolide, and para-dioxanone or with a prepolymer of the structure

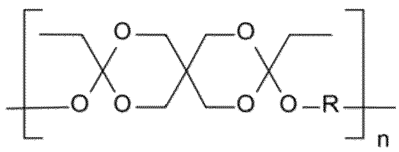,

wherein R are independently of each other straight, branched or cyclic C1 to C20 alkylene groups; with the proviso that for the use in a particle at least one of the at least one monomer(s) is selected from epsilon-caprolactone, delta-valerolactone, alpha-methylene-delta-valerolactone, aspartic acid, glutamic acid, glycolide, and para-dioxanone, if [A] does not comprise unit (b); wherein the prepolymer is preferably allowed to react in the presence of a catalyst.

All above-described embodiments for the block copolymer apply as well for the method.

Furthermore, the present invention refers to the use of a nanoparticle, comprising at least one block copolymer according to the present invention or at least one block copolymer obtained by the method according to the present invention.

In general any method of preparing particles is suitable and the skilled person knows how to obtain particles according to the present invention. In one embodiment the particles are obtained via a nanosonication process, a preferred process is for example disclosed in WO 2021/063813 A1.

In one embodiment the particle or nanoparticle according to the present invention further comprises at least one active agent and optionally at least one additive. The at least one active agent is preferably comprised in an effective amount.

A wide variety of active agents can be used with the particles described herein. In one embodiment, the active agent can be a releasable active agent, i.e., an active agent that can be released from the particle into adjacent tissues or fluids of a subject. In certain embodiments, the active agent can be in or on the particle.

Various forms of the active agent can be used, which are capable of being released from the particle into adjacent tissues or fluids. To that end, a liquid or solid active agent can be incorporated into the particles described herein. The active

agents are at least very slightly water soluble, and preferably moderately water soluble. The active agents can include salts of the active ingredient. As such, the active agents can be acidic, basic, or amphoteric salts. They can be nonionic molecules, polar molecules, or molecular complexes capable of hydrogen bonding. The active agent can be included in the compositions in the form of, for example, an uncharged molecule, a molecular complex, a salt, an ether, an ester, an amide, polymer drug conjugate, or other form to provide the effective biological or physiological activity.

Active agents, as used herein, include any molecule or compound capable of exerting a desired effect on a cell, tissue, organ, or subject. Such effects may be biological, physiological, or cosmetic. Active agents may be any type of molecule or compound, including e.g., nucleic acids, nucleic acid analogues, peptides and polypeptides, including, e.g., antibodies, such as, e.g., polyclonal antibodies, monoclonal antibodies, antibody fragments; humanized antibodies, recombinant antibodies, recombinant human antibodies, and Primatized™ antibodies, cytokines, growth factors, apoptotic factors, differentiation-inducing factors, cell surface receptors and their ligands; hormones; and small molecules, including small organic molecules or compounds.

Examples of active agents that incorporated into particles herein include, but are not limited to, peptides, proteins such as hormones, enzymes, antibodies, antibody fragments and the like, nucleic acids such as aptamers, iRNA, mRNA, DNA, RNA, antisense nucleic acid or the like, antisense nucleic acid analogs or the like, low-molecular weight compounds, or high- molecular-weight compounds. Active agents contemplated for use in the disclosed microparticles include anabolic agents, antacids, anti-asthmatic agents, anti-cholesterolemic and anti-lipid agents, anti-coagulants, anti-convulsants, anti-diarrheals, anti-emetics, anti- infective agents including antibacterial and antimicrobial agents, anti-inflammatory agents, anti-manic agents, antimetabolite agents, anti-nauseants, anti-neoplastic agents, anti-obesity agents, anti-pyretic and analgesic agents, anti-spasmodic agents, anti-thrombotic agents, antitussive agents, anti-uricemic agents, anti-vascular growth agents, anti-vascular endothelial growth agents, anti-anginal agents, antihistamines, appetite suppressants, biologicals, cerebral dilators, coronary dilators, bronchiodilators, cytotoxic agents, decongestants, diuretics, diagnostic agents, erythropoietic agents, expectorants, gastrointestinal sedatives, hyperglycemic agents, hypnotics, hypoglycemic agents, immunomodulating agents, ion exchange resins, laxatives, mineral supplements, mucolytic agents, neuromuscular drugs, peripheral vasodilators, psychotropics, sedatives, stimulants, thyroid and anti-thyroid agents, tissue growth agents, vascular growth agents, vascular endothelial growth agents, uterine relaxants, vitamins, or antigenic materials.

Other active agents include androgen inhibitors, polysaccharides, growth factors, hormones, anti-angiogenesis factors, dextromethorphan, dextromethorphan hydrobromide, noscapine, carbetapentane citrate, chlophedianol hydrochloride, chlorpheniramine maleate, phenindamine tartrate, pyrilamine maleate, doxylamine succinate, phenyltoloxamine citrate, phenylephrine hydrochloride, phenylpropanolamine hydrochloride, pseudoephedrine hydrochloride, ephedrine, codeine phosphate, codeine sulfate morphine, mineral supplements, cholestyramine, N-acetylprocainamide, acetaminophen, aspirin, ibuprofen, phenyl propanolamine hydrochloride, caffeine, guaifenesin, aluminum hydroxide, magnesium hydroxide, peptides, polypeptides, proteins, amino acids, hormones, interferons, cytokines, and vaccines.

Representative drugs that can be used as active agents in the particles include, but are not limited to, peptide drugs, protein drugs, desensitizing materials, antigens, anti-infective agents such as antibiotics, antimicrobial agents, antiviral, antibacterial, antiparasitic, antifungal substances and combination thereof, antiallergenics, androgenic steroids, decongestants, hypnotics, steroidal anti-inflammatory agents, anticholinergics, sympathomimetics, sedatives, miotics, psychic energizers, tranquilizers, vaccines, estrogens, progestational agents, humoral agents, prostaglandins, analgesics, antispasmodics, antimalarials, antihistamines, cardioactive agents, nonsteroidal anti-inflammatory agents, antiparkinsonian agents, antihypertensive agents, β-adrenergic blocking agents, nutritional agents, and the benzophenanthridine alkaloids. The agent can further be a substance capable of acting as a stimulant, sedative, hypnotic, analgesic, anticonvulsant, and the like.

The particle can comprise a large number of active agents either singly or in combination. Other active agents include but are not limited to analgesics such as acetaminophen, acetylsalicylic acid, and the like; anesthetics such as lidocaine, xylocaine, and the like; anorexics such as dexedrine, phendimetrazine tartrate, and the like; antiarthritics such as methylprednisolone, ibuprofen, and the like; antiasthmatics such as terbutaline sulfate, theophylline, ephedrine, and the like; antibiotics such as sulfisoxazole, penicillin G, ampicillin, cephalosporins, amikacin, gentamicin, tetracyclines, chloramphenicol, erythromycin, clindamycin, isoniazid, rifampin, and the like; antifungals such as amphotericin B, nystatin, ketoconazole, and the like; antivirals such as acyclovir, amantadine, and the like; anticancer agents such as cyclophosphamide, methotrexate, etretinate, and the like; anticoagulants such as heparin, warfarin, and the like; anticonvulsants such as phenytoin sodium, diazepam, and the like; antidepressants such as isocarboxazid, amoxapine, and the like; antihistamines such as diphenhydramine HCl, chlorpheniramine maleate, and the like; hormones such as insulin, progestins, estrogens, corticoids, glucocorticoids, androgens, and the like; tranquilizers such as thorazine, diazepam, chlorpromazine HCl, reserpine, chlordiazepoxide HCl, and the like; antispasmodics such as belladonna alkaloids, dicyclomine hydrochloride, and the like; vitamins and minerals such as essential amino acids, calcium, iron, potassium, zinc, vitamin B 12 , and the like; cardiovascular agents such as prazosin HCl, nitroglycerin, propranolol HCl, hydralazine HCl, pancrelipase, succinic acid dehydrogenase, and the like; peptides and proteins such as LHRH, somatostatin,

calcitonin, growth hormone, glucagon-like peptides, growth releasing factor, angiotensin, FSH, EGF, bone morphogenic protein (BMP), erythropoietin (EPO), interferon, interleukin, collagen, fibrinogen, insulin, Factor VIII, Factor IX, Enbrel®, Rituxam® , Herceptin , alpha- glucosidase, Cerazyme/Ceredose® , vasopressin, ACTH, human serum albumin, gamma globulin, structural proteins, blood product proteins, complex proteins, enzymes, antibodies, monoclonal antibodies, antibody fragments, and the like; prostaglandins; nucleic acids; carbohydrates; fats; narcotics such as morphine, codeine, and the like, psychotherapeutics; anti-malarias, L-dopa, diuretics such as furosemide, spironolactone, and the like; antiulcer drugs such as rantidine HCl, cimetidine HCl, and the like.

The active agent can also be an immunomodulator, including, for example, cytokines, interleukins, interferon, colony stimulating factor, tumor necrosis factor, and the like; allergens such as cat dander, birch pollen, house dust mite, grass pollen, and the like; antigens of bacterial organisms such as Streptococcus pneumoniae, Haemophilus influenzae, Staphylococcus aureus, Streptococcus pyrogenes, Corynebacterium diphteriae, Listeria monocytogenes, Bacillus anthracis, Clostridium tetani, Clostridium botulinum, Clostridium perfringens. Neisseria meningitides, Neisseria gonorrhoeae, Streptococcus mutans. Pseudomonas aeruginosa, Salmonella typhi, Haemophilus parainfluenzae, Bordetella pertussis, Francisella tularensis, Yersinia pestis, Vibrio cholerae, Legionella pneumophila, Mycobacterium tuberculosis, Mycobacterium leprae, Treponema pallidum, Leptspirosis interrogans, Borrelia burgdorferi, Campylobacter jejuni, and the like; antigens of such viruses as smallpox, influenza A and B, respiratory syncytial, parainfluenza, measles, HIV, SARS, varicella-zoster, herpes simplex 1 and 2, cytomeglavirus, Epstein-Barr, rotavirus, rhinovirus, adenovirus, papillomavirus, poliovirus, mumps, rabies, rubella, coxsackieviruses, equine encephalitis, Japanese encephalitis, yellow fever, Rift Valley fever, lymphocytic choriomeningitis, hepatitis B, and the like; antigens of such fungal, protozoan, and parasitic organisms such as Cryptococcuc neoformans, Histoplasma capsulatum, Candida albicans, Candida tropicalis, Nocardia asteroids, Rickettsia ricketsii, Rickettsia typhi, Mycoplasma pneumoniae, Chlamyda psittaci, Chlamydia trachomatis, Plasmodium falciparum, Trypanasoma brucei, Entamoeba histolytica, Toxoplasma gondii, Trichomonas vaginalis, Schistosoma mansoni, and the like. These antigens may be in the form of whole killed organisms, peptides, proteins, glycoproteins, carbohydrates, or combinations thereof.

In a further specific aspect, the active agent comprises an antibiotic. The antibiotic can be, for example, one or more of Amikacin, Gentamicin, Kanamycin, Neomycin, Netilmicin, Streptomycin, Tobramycin, Paromomycin, Ansamycins, Geldanamycin, Herbimycin, Carbacephem, Loracarbef, Carbapenems, Ertapenem, Doripenem, Imipenem/Cilastatin, Meropenem, Cephalosporins (First generation), Cefadroxil, Cefazolin, Cefalotin or Cefalothin, Cefalexin, Cephalosporins (Second generation), Cefaclor, Cefamandole, Cefoxitin, Cefprozil, Cefuroxime, Cephalosporins (Third generation), Cefixime, Cefdinir, Cefditoren, Cefoperazone, Cefotaxime, Cefpodoxime, Ceftazidime, Ceftibuten, Ceftizoxime, Ceftriaxone, Cephalosporins (Fourth generation), Cefepime, Cephalosporins (Fifth generation), Ceftobiprole, Glycopeptides, Teicoplanin, Vancomycin, Macrolides, Azithromycin, Clarithromycin, Dirithromycin, Erythromycin, Roxithromycin, Troleandomycin, Telithromycin, Spectinomycin, Monobactams, Aztreonam, Penicillins, Amoxicillin, Ampicillin, Azlocillin, Carbenicillin, Cloxacillin, Dicloxacillin, Flucloxacillin, Mezlocillin, Meticillin, Nafcillin, Oxacillin, Penicillin, Piperacillin, Ticarcillin, Polypeptides, Bacitracin, Colistin, Polymyxin B, Quinolones, Ciprofloxacin, Enoxacin, Gatifloxacin, Levofloxacin, Lomefloxacin, Moxifloxacin, Norfloxacin, Ofloxacin, Trovafloxacin, Sulfonamides, Mafenide, Prontosil (archaic), Sulfacetamide, Sulfamethizole, Sulfanilimide (archaic), Sulfasalazine, Sulfisoxazole, Trimethoprim, Trimethoprim-Sulfamethoxazole (Co- trimoxazole) (TMP-SMX), Tetracyclines, including Demeclocycline, Doxycycline, Minocycline, Oxytetracycline, Tetracycline, and others; Arsphenamine, Chloramphenicol, Clindamycin, Lincomycin, Ethambutol, Fosfomycin, Fusidic acid, Furazolidone, Isoniazid, Linezolid, Metronidazole, Mupirocin, Nitrofurantoin, Platensimycin, Pyrazinamide, Quinupristin/Dalfopristin, Rifampicin (Rifampin in U.S.), Tinidazole, or a combination thereof. In one aspect, the active agent can be a combination of Rifampicin (Rifampin in U.S.) and Minocycline.

In one embodiment, the active agent is a therapeutic agent, or a salt or derivative thereof. Therapeutic agent derivatives may be therapeutically active themselves or they may be prodrugs, which become active upon further modification.

In one embodiment, therapeutic agents include any therapeutically effective agent or drug, such as anti-inflammatory compounds, anti-depressants, stimulants, analgesics, antibiotics, birth control medication, antipyretics, vasodilators, anti-angiogenics, cytovascular agents, signal transduction inhibitors, cardiovascular drugs, e.g., anti-arrhythmic agents, vasoconstrictors, hormones, and steroids.

In one embodiment the therapeutic agent is an oncology drug, which may also be referred to as an antitumor drug, an anti-cancer drug, a tumor drug, an antineoplastic agent, or the like. Examples of oncology drugs that may be used according to the invention include, but are not limited to, adriamycin, alkeran, allopurinol, altretamine, amifostine, anastrozole, arsenic trioxide, azathioprine, bexarotene, biCNU, bleomycin, busulfan intravenous, busulfan oral, capecitabine (Xeloda), carboplatin, carmustine, CCNU, celecoxib, chlorambucil, cisplatin, cladribine, cyclosporin A, cytarabine, cytosine arabinoside, daunorubicin, Cytoxan, daunorubicin, dexamethasone, dexrazoxane, dodetaxel, doxorubicin, doxorubicin, DTIC, epirubicin, estramustine, etoposide phosphate, etoposide and VP-16, exemestane, FK506, fludarabine, fluorouracil, 5-FU, gemcitabine (Gemzar), gemtuzumab-ozogamicin, goserelin acetate, hydrea, hydroxyurea, idarubicin, ifosfamide, imatinib mesylate, interferon, irinotecan (Camptostar, CPT-111), letrozole, leucovorin, leustatin, leuprolide, levamisole, litretinoin, megastrol, melphalan, L-PAM, mesna, methotrexate, methoxsalen, mithramycin, mitomycin, mitoxantrone, nitrogen mustard, paclitaxel, pamidronate, Pegademase, pentostatin, porfimer sodium, prednisone, rituxan, streptozocin, STI-571, tamoxifen, taxotere, temozolamide, teniposide, VM-26, topotecan (Hycamtin), toremifene, tretinoin, ATRA, valrubicin, velban, vinblastine, vincristine, VP16, and vinorelbine. Other examples of oncology drugs that may be used according to the invention are ellipticin and ellipticin analogs or derivatives, epothilones, intracellular kinase inhibitors and camptothecins.

In an embodiment the at least one active agent is selected from the group consisting of proteins, peptides, carbohydrates, nucleic acids and nucleic acid analogues, organic molecules having a molecular weight of up to 1000 g/mol and combinations thereof.

Any known protein is in general suitable. Exemplarily proteins include glycoproteins and apolipoproteins. As used herein, the term "apolipoprotein" or "lipoprotein" refers to apolipoproteins known to those of skill in the art and variants and fragments thereof and to apolipoprotein agonists, analogues or fragments thereof as well as chimeric construction of an apolipoprotein. Apolipoproteins utilized in the invention also include recombinant, synthetic, semi- synthetic or purified apolipoproteins.

Any known peptide is in general suitable. The term peptide according to the present invention includes peptidomimetic. The peptide or peptidomimetic can be about 5 to 50 amino acids long, e.g., about 5, 10, 15, 20, 25, 30, 35, 40, 45, or 50 amino acids long. A "cell permeation peptide" is capable of permeating a cell, e.g., a microbial cell, such as a bacterial or fungal cell, or a mammalian cell, such as a human cell. A microbial cell-permeating peptide can be, for example, an α-helical linear peptide (e.g., LL-37 or Ceropin PI), a disulfide bond-containing peptide (e.g., α-defensin, β-defensin or bactenecin), or a peptide containing only one or two dominating amino acids (e.g., PR-39 or indolicidin). A cell permeation peptide can also include a nuclear localization signal (NLS). For example, a cell permeation peptide can be a bipartite amphipathic peptide, such as MPG, which is derived from the fusion peptide domain of HIV-1 gp41 and the NLS of SV40 large T.

In one embodiment, a targeting peptide tethered to an iRNA agent and/or the carrier oligomer can be an amphipathic α-helical peptide.

Peptide and peptidomimetic ligands include those having naturally occurring or modified peptides, e.g., D- or L-peptides; α-, β-, or γ-peptides; N-methyl peptides; azapeptides; peptides having one or more amide, i.e., peptide, linkages replaced with one or more urea, thiourea, carbamate, or sulfonyl urea linkages; or cyclic peptides.

Any known carbohydrate is in general suitable as active agent. Exemplarily carbohydrates include dextran, pullulan, chitin, chitosan, inulin, cyclodextrin or hyaluronic acid.

In one embodiment the active agent is a nucleic acid or nucleic acid analogue, including, e.g., siRNA molecules, mRNA molecules, plasmids, micro RNA, antagomirs, aptamers, and ribozymes.

Nucleic acids and nucleic acid analogues include polymers containing at least two deoxyribonucleotides or ribonucleotides in either single- or double- or triple-stranded form and includes DNA, RNA, and hybrids thereof. DNA may be in the form of linear DNA, circular DNA, plasmid DNA (pDNA), antisense molecules, PCR products, or vectors. RNA may be in the form of chemically modified or unmodified messenger RNA (mRNA), self-amplifying RNA (saRNA), circular RNA (circRNA) comprising at least one coding sequence, small hairpin RNA (shRNA), small interfering RNA (siRNA), micro RNA (miRNA), dicer substrate RNA, antisense oligonucleotide (ASO), transfer RNA (tRNA), single guide RNA (sgRNA) or viral RNA (vRNA) and combinations thereof. The nucleic acids may include one or more oligonucleotide modification.

In one embodiment the at least one active agent is an organic molecule having a molecular weight up to 1000 g/mol, also referred to as small molecule in the pharmaceutical field, preferably the organic molecule is selected from paclitaxel, doxorubicin, irinotecan, vincristine and oxaliplatin.

Additionally, at least one of the following additives can be further present in the composition: preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; carbohydrates including monosaccharides, disaccharides, and other sugar compounds like glucose, mannose, or dextrins; chelating agents such as EDTA; sugars such as sucrose, mannitol, trehalose or sorbitol; salt-forming counter-ions such as sodium ion; metal complexes (e.g., Zn-protein complexes), vehicles, binders, disintegrants, immunological adjuvants like a cell penetrating peptide, for example human lactoferrin protein or a fragment thereof, Tat, Ant, Rev, FHV, HSV-1 protein VP22, C6, C6M1, PF20, NAP, POD, polyarginine, polylysine, PTD-5, Transportan, MAP, TP10, Pep-7, Azurin p18, Azurin p28, hCT18-32, Bac 7, CTP, K5-FGF, HAP-1, 293P-1, KALA, GALA, LAH4-L1, Melittin, Penetratin, EB1, MPG, CADY, Pep4, preferably a human lactoferrin protein or a fragment thereof, fillers (diluents), lubricants, glidants (flow enhancers), compression aids, colors, sweeteners, suspending/dispersing agents, film formers/coatings, flavors, printing inks.

In one embodiment the at least one additive is present in 0.0001 to 5 wt.-%, preferably 0.01 to 4 wt.-%, more

preferably 0.1 to 3 wt.-% based on the total weight of the particle.

In one embodiment the nanoparticle has a mean diameter of 20 to 500 nm, preferably 30 to 400, more preferably 50 to 250 nm, preferably measured via dynamic light scattering, more preferably according to ISO 22412:2017. The measurements can be conducted with a Malvern Zetasizer NanoZS.

In one embodiment the particle can comprise 1%, 5%, 10%, 20%, 30%, 40%, 50%, 60%, 70%, 80%, 90%, 95% by weight active agent, relative to the total weight of the particle, including any range between the disclosed percentages.

The particle used according to the present invention may be prepared by a method comprising or consisting of the steps:

i) dissolving at least one block copolymer according to any of claims 1 to 7 or a block copolymer obtained by the method according to claim 8 in an organic solvent in order to obtain a dispersed phase;
ii) dissolving at least one stabilizer, such as polyvinyl alcohol (PVA), in water, preferably deionized water, in order to obtain a continuous phase;
iii simultaneously pumping the dispersed phase and the continuous phase into a sonicator in order to obtain an emulsion;
iv) extracting the emulsion, inline, into a stream of water, preferably deionized water in order to obtain the particles.

This method provides an aqueous solution comprising at least one block copolymer according to the present invention or a block copolymer obtained by the method according to the present invention.

The particles may be used for the treatment of an illness in humans. The particles may also be used for the treatment of an illness in mammals. The particles of the present invention may be administered in a therapeutically effective amount, which will vary depending upon a variety of factors including the activity of the specific therapeutic agent employed; the metabolic stability and length of action of the therapeutic agent; the age, body weight, general health, sex, and diet of the patient; the mode and time of administration; the rate of excretion; the drug combination; the severity of the particular disorder or condition; and the subject undergoing therapy.

In a preferred embodiment the particle of the present invention is part of a composition. The composition may be a pharmaceutical composition or a non-pharmaceutical composition. In one embodiment the present invention concerns a use, wherein the at least one block copolymer is used in a particle and wherein the particle is part of a composition, preferably a pharmaceutical composition.

The medical device may comprise at least one block copolymer according to the present invention or at least one block copolymer obtained by the aforementioned method. The medical device may comprise at least one particle, which comprises at least one block copolymer. In one embodiment the present invention concerns a use, wherein the at least one block copolymer is used in a particle and wherein the particle is used in a medical device. In one embodiment the medical device is an implantable medical device. In one embodiment the medical device is selected from the group consisting of catheters, sutures, staples, surgical clips, and implants.

It is well known to the skilled person in the medical device field how to obtain a medical device from a suitable block copolymer, like the block copolymer according to the present invention. For example Manavitehrani, Iman et al. "Biomedical Applications of Biodegradable Polyesters." Polymers vol. 8,1 20. discloses methods to obtain a medical device.

## Examples

### Reagents and chemicals

Reagents and chemicals were purchased from TCI (Tokyo, Japan), Thermo Fisher Scientific (Waltham, MA, USA), Carl Roth GmbH (Karlsruhe, Germany) and Merck KGaA (Darmstadt, Germany) unless otherwise noted. Ethylene oxide (EO) was obtained from Air Liquide (Paris, France). Tetrahydrofuran (THF) was flashed over basic aluminum oxide before usage. Glycidyl methyl ether (GME) was dried over Calcium hydride ($CaH_2$) and cryo-transferred before polymerization. mPEOSk was obtained from Pharmicell Co. Ltd (Seoul, South Korea).

### Synthesis of poly(ethylene oxide)s

**Example 1:** Synthesis of glycidyl methyl ether (GME)

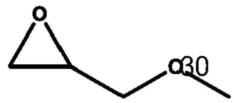

1-Chloro-3-methoxy-propan-2-ol (50.0 g, 401 mmol) was added to a flask equipped with a magnetic stirrer and cooled with an ice bath. Finely grounded sodium hydroxide (NaOH, 20.9 g, 522 mmol) was added in portions under stirring. After complete reaction (TLC control) the crude product was cryo-transferred in vacuo from the reaction flask and dried over $CaH_2$ under cooling with an ice bath. After an additional cryo-transfer and filtration step, GME (30.1 g, 85%) was obtained as a colorless liquid.

**Example 2:** Synthesis of 1-methoxy-3-(2-methoxyethoxy)propan-2-ol

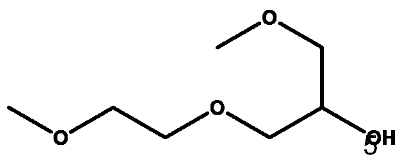

Ethylene oxide monomethyl ether (8.64 g, 9.00 mL, 113 mmol) was added to a flask equipped with a reflux condenser. NaOH solution (19 M, 3 mL) was added under stirring and the resulting solution was heated to 55 °C. GME (5.00 g, 5.10 mL, 56.7 mmol) was added and the solution was stirred overnight. The solution was cooled to room temperature and extracted with dichloromethane (DCM, 50 mL) three times. The combined organic phases were dried over magnesium sulphate ($MgSO_4$). After filtration, the solvent was evaporated under reduced pressure. 1-Methoxy-3-(2-methoxyethoxy)propan-2-ol (3.74 g, 40%) was obtained as a colorless liquid after fractional distillation of the residue.

**Example 3:** Synthesis of mP($EO_{67}$-*co*-$GME_{38}$)

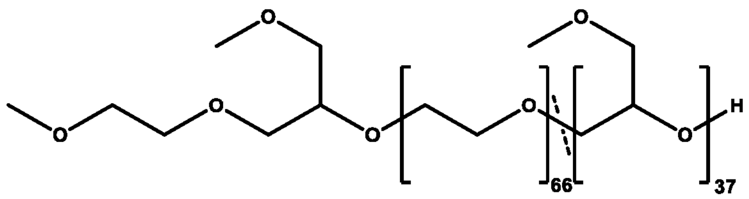

KOtBu (98.0 mg, 877 μmol) was dissolved in stabilizer-free THF and small quantities of Millipore water and transferred into a flame-dried and argon flushed flask equipped with a Teflon stopcock and a septum. 1-Methoxy-3-(2-methoxyethoxy)propan-2-ol (147 mg, 895 μmol) was dissolved in benzene and transferred into the flask. High vacuum was applied to the flask and the solvents were removed under high vacuum. The resulting initiator salt was dried under high vacuum at 55 °C overnight. The residue was dissolved in dry DMSO (34 mL). After freezing the resulting solution at -80 °C, GME (1.58 g, 1.61 mL, 17.9 mmol) was added to the flask via syringe. EO (1.40 g, 1.44 mL, 31.8 mmol) was added to the flask via cryo-transfer from a graduated ampoule. The cooling bath was removed, and the reaction mixture was stirred for 1 d at 30 °C under high vacuum. The solution was cooled to -80 °C and GME (1.58 g, 1.61 mL, 17.9 mmol) was added to the flask via syringe. EO (1.40 g, 1.44 mL, 31.8 mmol) was added to the flask via cryo-transfer from a graduated ampoule. The cooling bath was removed, and the reaction mixture was stirred for 1 d at 30 °C under high vacuum. The solvent was evaporated under high vacuum at 50 °C. Millipore water (100 mL) and acidic ion-exchange resin (DOWEX) (500 mg) were added to the residue. The resulting suspension was stirred overnight. The suspension was filtered, and the resulting solution was lyophilized. The residue was dissolved in diethyl ether (400 mL). The resulting suspension was filtered, and the organic phase was dried over $MgSO_4$. After a filtration step, the solvent was evaporated to yield mP($EO_{67}$-*co*-$GME_{38}$) (96%, PDI = 1.08) as a viscous liquid.

Copolymers were characterized by nuclear magnetic resonance (NMR) spectroscopy, size exclusion chromatography (SEC), and matrix-assisted laser-desorption-ionization time of flight mass spectroscopy (MALDI-TOF):

$^1$H NMR spectra were recorded on a Bruker (Billerica, MA, U.S.) III HD 300 spectrometer with 300 MHz and referenced internally to residual proton signals of the deuterated solvent.

SEC: $M_w$, $M_n$ and dispersities ($M_w/M_n$ = D) of all samples were determined from the corresponding size exclusion chromatograms (refractive index (RI) detector, calibration with PEO standards). SEC measurements were performed with dimethylformamide (DMF with 1 g/L lithium bromide (LiBr)) as the mobile phase (flow rate 1 mL/min) on poly(2-hydroxyethylmethacrylat) (PHEMA) 300/100/40 columns at 50 °C. Polymer concentrations were 1 mg/mL. Calibration was carried out using PEO standards (from Polymer Standard Service, Mainz, Germany).

MALDI-TOF MS measurements were carried out at a Bruker autoflex maX MALDI-TOF/TOF. The potassium salt of trifluoroacetic acid and trans 2 [3-(4-tert-butylphenyl)-2-methyl-2-propenylidene]malononitrile (DCTB) were used as ionization salt and matrix, respectively. Data was used to calculate $M_n$ of the polymer and degree of polymerization (DP) for the respective comonomers.

Table 1: Characterization of mP($EO_{67}$-*co*-$GME_{38}$)

| | |
|---|---|
| $DP_{n,GME,MALDI}$ | 38 |
| $DP_{n,EO,MALDI}$ | 67 |
| $M_{n,SEC}$ [kDa] | 5.2 |
| $M_{n,MALDI}$ [kDa] | 6.3 |
| $Đ_{SEC,RI}$ | 1.08 |

**Example 4:** Determination of immunogenicity of poly(ethylene oxide)s by competitive enzyme-linked immunosorbent assay (ELISA)

The interaction of mP($EO_{67}$-*co*-$GME_{38}$) from Example 3 and mPEOSk with anti-PEG antibodies (APAs) was evaluated by a competitive PEO ELISA kit using a murine monoclonal, horseradish peroxidase conjugated anti PEO antibody (HRP anti-PEO, Life diagnostics, West Chester, PA, USA). Samples of concentrations ranging from 0 to 4600 $\mu$g $mL^{-1}$ were prepared in dilution buffer. 50 $\mu$L of each prepared sample was dispensed to a PEO pre-coated 96-well plate and 50 $\mu$L of HRP anti-PEO was added to each well. The solutions were incubated for 1 h at 25 °C with a micro-plate shaker and then washed six times with 400 $\mu$L of wash buffer per each well. After removal of residual droplets, 100 $\mu$l of 3,3',5,51-Tetramethylbiphenyl-4,4'-diamine was added to each well and the solutions were mixed on a micro-plate shaker for 20 min. The reaction was stopped by addition of 100 $\mu$L of stop solution and the absorbance at 450 nm was read within 5 min.

Analysis of ELISA Data: The determined absorbance values were normalized to visualize the percent of maximal binding. The sample concentrations were transformed to a function of $\log_{10}$. The sigmoidal fits were calculated using the following equation with a representing the upper, b the lower limit, c the inflection point and d the hill slope.

$$y = a + (b-a) / 1 + 10^{(c-x)*d}$$

**Fig. 1** shows the ELISA test result of mP($EO_{67}$-*co*-$GME_{38}$) from Example 3 and mPEO5k. Figure shows the function of the normalized absorption at a wavelength of 450 nm versus the $\log_{10}$ function of the polymer concentration in nanograms per mL, therefore illustrating the APA interaction with the investigated polymers at various concentrations. Interaction of the polymers with the APA reduces the adsorption. The ELISA data shows a strong influence of the methoxy methyl side chains on the interaction of polymer with the APA (it is important to note that the x-axis has a logarithmic scale). With the incorporation of sterically demanding methoxy methyl side groups into the polyether structure, significantly higher polymer concentrations are necessary to observe interactions between the polymer and the APA.

**Synthesis of block copolymers**

**Example 5:** Synthesis of mP($EO_{67}$-co-$GME_{38}$)-PDLA15k block copolymer

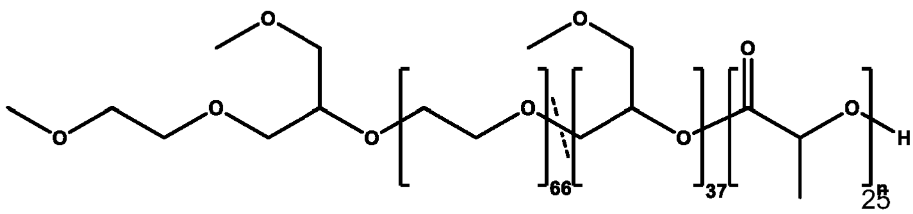

mP($EO_{67}$-*co*-$GME_{38}$)-PDLA15k was synthesized via ring-opening polymerization (ROP) of D,L-Lactide at 140 °C using m($EO_{67}$-co-$GME_{38}$) from Example 3 as the macroinitiator. Briefly, 0.889 g (0.13 mmol) mP($EO_{67}$-*co*-$GME_{38}$) and 2.113 g (41.66 mmol) D,L-Lactide were combined in a 7.4 mL septum sealed vial and melted at 140 °C under $N_2$ utilizing a block heater. Simultaneously, a second polymerization (comparative example) was prepared as a control using mPEO5k as the macroinitiator, targeting an identical lactide/initiator mol ratio. Once molten, 75 μL of a 100 mg/mL $Sn(Oct)_2$ solution in toluene was added to each vial to afford a catalyst concentration of 2.5 ppt. To promote mixing, the vials were manually inverted approximately every 5 min for the first 30 min of the polymerization. The reactions were allowed to proceed for 18 h, after which vacuum stripping was performed for an additional 2 h at 140 °C. The products were recovered by removing the vials from the heating block without further purification.

Polymers were characterized via $^1H$ NMR using a Varian (Palo Alto, CA, U.S.) INOVA 400 MHz NMR spectrometer in $CDCl_3$ using a delay time of 5 s. rPEG copolymer composition was determined by comparing the p(D,L-Lactide) methine resonances at 5.20 ppm and rPEG methylene and methine resonances at 3.40-3.85 ppm to the rPEG methoxy resonances at 3.38 ppm. mPEO copolymer compositions were determined by comparing the p(D,L-Lactide) methine resonances at 5.20 ppm and monomethoxy PEO (mPEO) methylene resonances at 3.65 ppm to the mPEO methoxy end group resonance at 3.38 ppm.

Table 2: Characterization of block copolymer molecular weight by $^1H$ NMR

| *Entry (Example)* | *Sample* | $M_{n,PEO,NMR}$ [kDa] | $M_{n,PDLA,NMR}$ [kDa] | $M_{n,Copolymer,NMR}$ [kDa] |
|---|---|---|---|---|
| a(5) | mP($EO_{67\text{-}CO}$-$GME_{38}$)-PDLA15k | 7.1 | 15.4 | 22.6 |
| b(5)* | mPEO5k-PDLA15k | 4.9 | 14.6 | 19.5 |

* Comparative Example

Thermal analysis of polymers was carried out by differential scanning calorimetry (DSC) using a TA Instruments (Waters Corp., New Castle, DE, US) DSC 250 according to ASTM D 3418. The thermal history of the samples was excluded via three cooling and three heating cycles:

Cooling: Ramp 10 K/min to -75 °C
Isothermal for 1 min
Heating: Ramp 10 K/min to 200 °C
Isothermal for 1 min
Cooling: Ramp 5 K/min to -75 °C
Isothermal for 1 min
Heating: Ramp 5 K/min to 200 °C
Isothermal for 1 min
Cooling: Ramp 5 K/min to -75 °C
Isothermal for 1 min
Heating: Ramp 10 K/min to 200 °C

For each sample, the glass transition temperature ($T_g$) was obtained from the third heating curve.

**Fig. 2** shows the thermogram of polymers a(5) and b(5). a(5) exhibits one $T_g$ at -1 °C. This indicates miscibility of the two polymer phases. b(5) exhibits two glass transition temperatures: -3 °C for the PEO and 48 °C for the PDLA segment. This indicates phase separation of the two polymer phases.

**Preparation of nanoparticles (NPs)**

**Example 6:** Preparation of NPs by inline sonication/emulsion process

Polymer solutions (dispersed phase) were prepared at 10 %(w/w) in ethyl acetate. Samples were placed into a water bath at 30 °C for 1 min to ensure entire dissolution. Polymer solutions were filtered via a 0.45 μm PTFE syringe filter. The continuous phase consisted of a 1%(w/w) polyvinyl alcohol (PVA) solution (PVA 5-88, Merck KGaA Darmstadt, Germany). The dispersed phase was pumped into the sonicator via a syringe and syringe pump and the continuous phase was pumped via an ISCO piston pump at 4 mL/min and 12 mL/min, respectively. The sonicator was operated at 100% amplitude. The emulsion was continuously extracted, inline, into a stream of deionized water pumped via a peristaltic pump at 240 mL/min. The resultant nanosuspension was collected in a beaker and stirred openly in the fume hood for 10 min before use.

Mean diameter (z-ave.) and zeta potential (z-pot.) of NPs were measured with a Zetasizer NanoZS from Malvern Instruments GmbH (Herrenberg, Germany). A DTS 1070 clear disposable folded capillary cell from Malvern Panalytical GmbH (Kassel, Germany) was used. Z-ave. and the width of the fitted Gaussian distribution, which is displayed as the polydispersity index (PDI), were calculated from data of at least 10 runs. Measurements were carried out in triplicate and results are presented as mean average ± standard deviation.

Table 3: Z-ave., PDI and z-pot. of NPs

| *Entry (Example)* | *Polymer used* | *Z-ave.* [nm] | *PDI* | *z-pot. [mV]* |
|---|---|---|---|---|
| a(5) | mP($EO_{67}$-*co*-$GME_{38}$)-PDLA15k | 147.6±2.1 | 0.226±0.008 | -6.6±0.1 |
| b(5)* | mPEO5k-PDLA15k | 125.2±0.9 | 0.144±0.008 | -11.4±1.0 |

* Comparative Example

**Conclusion**

A new class of block copolymers comprising a PEO with C1 to C3-alkyloxymethyl side chains block and a hydrophobic block. mP(EO-*co*-GME) was manufactured by anionic ring opening polymerization (AROP). ELISA assay revealed significantly weaker binding affinity of mP(EO-co-GME) towards APAs compared to PEO. mP(EO-co-GME) was used as macroinitiator for the ROP of D,L-Lactide. DSC revealed different polymer phase separation and thermal properties of the mP(EO-co-GME)-PDLA block copolymer compared to the mPEO-PDLA block copolymer: mP(EO-co-GME)-PDLA exhibits one $T_g$, indicating miscibility of the two polymer phases. mPEO-PDLA exhibits two $T_g$s, indicating phase separation. Block copolymers were used for the preparation of nanoparticles by inline sonication/emulsion. Both polymers resulted in monomodally distributed nanosuspensions. Z-ave. diameters for the rPEG and PEO block copolymers were 147.6±2.1 and 125.2±0.9 nm, respectively.

**Claims**

1. Use of at least one block copolymer in a particle or in a medical device, wherein the at least one block copolymer is obtained by reacting

at least one monomer, preferably one or two monomers, selected from L-lactide, D-lactide, DL-lactide, epsilon-caprolactone, trimethylene carbonate, delta-valerolactone, alpha-methylene-delta-valerolactone, aspartic acid, glutamic acid, glycolide, and para-dioxanone, or by reacting the prepolymer of the structure:

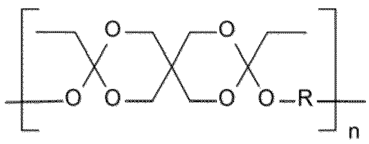

,

wherein R are independently of each other straight, branched or cyclic C1 to C20 alkylene groups; with a prepolymer H-[A]-$R^1$;
wherein [A] is a polyoxyalkylene group comprising at least one unit

(a)

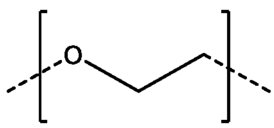

and at least one unit selected from the group of
(b)

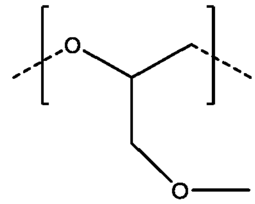
,

(c)

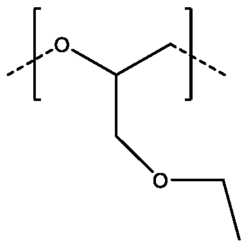
,

(d)

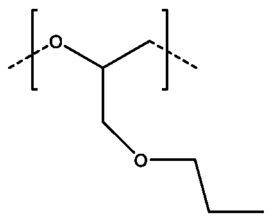
,

and
(e)

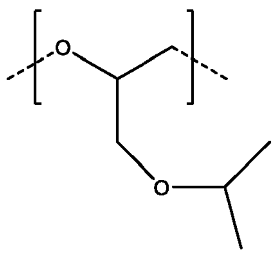
;

wherein

$R^1$ is selected from -H, -OH, -SH, $-NH_2$, $-NHR^2$, $-NR^3R^4$, $-OR^5$, $-SR^5$ or linear, branched or cyclic alkyl groups having up to 20 carbon atoms; and wherein
$R^2$ to $R^5$ are independently selected from linear, branched or cyclic alkyl groups having up to 20 carbon atoms, in which up to 5 carbon atoms can be substituted with an oxygen or a sulfur atom; with the proviso that for the use in a particle at least one of the at least one monomer(s) is selected from epsilon-caprolactone, delta-valerolactone, alpha-methylene-delta-valerolactone, aspartic acid, glutamic acid, glycolide, and para-dioxanone, if [A] does not comprise unit (b).

**2.** The use according to claim 1, wherein the block copolymer has a number average molecular weight of 1,200 to 600,000 g/mol, preferably 2,000 to 250,000 g/mol, more preferably 5,000 to 100,000 g/mol, preferably determined via size exclusion chromatography using a refractive index (RI) detector and polyethylene oxide standards.

**3.** The use according to any of the preceding claims, wherein the reaction is performed in the presence of a catalyst.

4. The use according to claim 3, wherein the catalyst is a ring opening catalyst or a polycondensation catalyst, preferably the ring opening catalyst is a tin catalyst, more preferably tin (II) 2-ethylhexanoate, or preferably the polycondensation catalyst is an organic acid, more preferably p-toluenesulfonic acid.

5. The use according to any of the preceding claims, wherein the at least one monomer, preferably one or two monomers, is selected from L-lactide, D-lactide, DL-lactide, epsilon-caprolactone, trimethylene carbonate, delta-valerolactone, alpha-methylene-delta-valerolactone, glycolide, and para-dioxanone, preferably from L-lactide, D-lactide, DL-lactide, epsilon-caprolactone, and glycolide, more preferably from L-lactide, D-lactide, DL-lactide, and glycolide.

6. The use according to any of the preceding claims, wherein H-[A]-$R^1$ has a number average molecular weight of 200 to 200,000 g/mol, preferably 500 to 100,000 g/mol, more preferably 1,000 to 50,000 g/mol, preferably determined via size exclusion chromatography using a refractive index (RI) detector and polyethylene oxide standards.

7. The use according to any of the preceding claims wherein the polyoxyalkylene group [A] comprises or consist of at least one unit

(a)

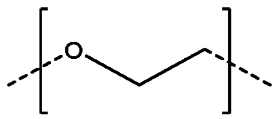

and at least one unit
(b)

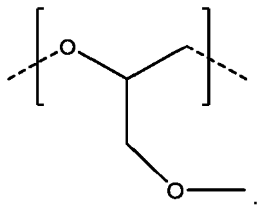

8. The use according to any of the preceding claims, wherein $R^1$ is -$OR^5$, preferably wherein $R^5$ is selected from linear, branched or cyclic alkyl groups having up to 20 carbon atoms, in which up to 5 carbon atoms can be substituted with an oxygen atom.

9. The use according to any of the preceding claims, wherein the particle is a nanoparticle.

10. The use according to any of the preceding claims, wherein the particle comprises at least one active agent.

11. The use according to any of the preceding claims, wherein the particle comprises at least one additive.

12. The use according to any of the preceding claims, wherein the particle has a mean diameter of 20 to 500 nm, preferably 30 to 400, more preferably 50 to 250 nm, preferably measured via dynamic light scattering, more preferably according to ISO 22412:2017.

13. The use according to any of the preceding claims, wherein the at least one block copolymer is used in a particle and wherein the particle is used in a medical device.

14. The use according to any of the preceding claims, wherein the at least one block copolymer is used in a particle and wherein the particle is part of a composition, preferably a pharmaceutical composition.

15. The use according to any of the preceding claims, with the proviso that if the at least one monomer is two monomers and [A] does not comprise at least one unit (b), the two monomers are not lactide and glycolide.

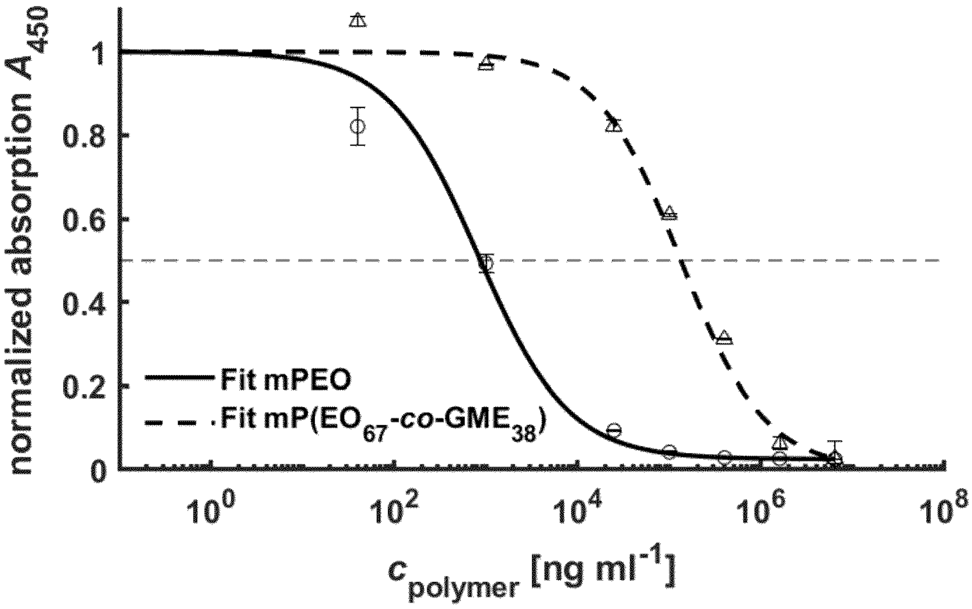
normalized absorption $A_{450}$
1
0.8
0.6
0.4
0.2
0
Fit mPEO
Fit mP(EO$_{67}$-*co*-GME$_{38}$)
$10^0$
$10^2$
$10^4$
$10^6$
$10^8$
$c_{polymer}$ [ng ml$^{-1}$]

Fig. 1

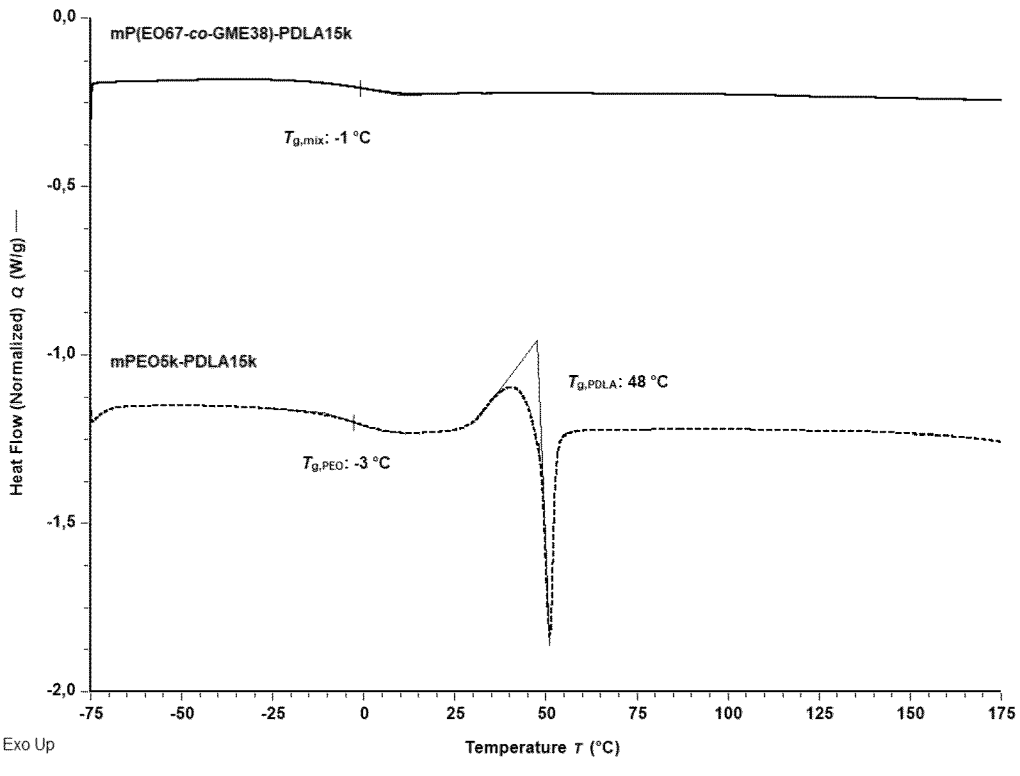
mP(EO67-*co*-GME38)-PDLA15k
$T_{g,mix}$: -1 °C
mPEO5k-PDLA15k
$T_{g,PDLA}$: 48 °C
$T_{g,PEO}$: -3 °C
Heat Flow (Normalized) Q (W/g)
0,0
-0,5
-1,0
-1,5
-2,0
-75
-50
-25
0
25
50
75
100
125
150
175
Exo Up
Temperature T (°C)

Fig. 2

Europäisches Patentamt
European Patent Office
Office européen des brevets

# EUROPEAN SEARCH REPORT

Application Number
EP 24 18 4969

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2022/238532 A1 (UNIV MAINZ JOHANNES GUTENBERG [DE]) 17 November 2022 (2022-11-17) * pages 22,40, paragraph 1 - page 28; claims 1,15; figure 19; example 13; table 1 * ----- | 1-15 | INV. C08G63/08 |
| Y,D | WO 2021/063813 A1 (EVONIK OPERATIONS GMBH [DE]) 8 April 2021 (2021-04-08) * example 1 * ----- | 1-15 | |

TECHNICAL FIELDS SEARCHED (IPC)

C08G
C09J
A61K

The present search report has been drawn up for all claims

1

| Place of search | Date of completion of the search | Examiner |
|---|---|---|
| The Hague | 25 November 2024 | Adigbli, Francis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 18 4969

This annex lists the patent family members relating to the patent documents cited in the above-mentioned European search report. The members are as contained in the European Patent Office EDP file on
The European Patent Office is in no way liable for these particulars which are merely given for the purpose of information.

25-11-2024

| Patent document cited in search report | Publication date | Patent family member(s) | Publication date |
|---|---|---|---|
| WO 2022238532 A1 | 17-11-2022 | CA 3218006 A1 | 17-11-2022 |
| | | EP 4089133 A1 | 16-11-2022 |
| | | EP 4337714 A1 | 20-03-2024 |
| | | JP 2024518983 A | 08-05-2024 |
| | | TW 202311354 A | 16-03-2023 |
| | | US 2024317934 A1 | 26-09-2024 |
| | | WO 2022238532 A1 | 17-11-2022 |
| WO 2021063813 A1 | 08-04-2021 | AU 2020360526 A1 | 26-05-2022 |
| | | BR 112022005352 A2 | 14-06-2022 |
| | | CA 3152315 A1 | 08-04-2021 |
| | | CN 114555064 A | 27-05-2022 |
| | | DK 3962464 T3 | 19-06-2023 |
| | | EP 3962464 A1 | 09-03-2022 |
| | | ES 2944558 T3 | 22-06-2023 |
| | | FI 3962464 T3 | 24-05-2023 |
| | | IL 291794 A | 01-06-2022 |
| | | JP 2022550829 A | 05-12-2022 |
| | | KR 20220074910 A | 03-06-2022 |
| | | US 2022354803 A1 | 10-11-2022 |
| | | WO 2021063813 A1 | 08-04-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022152835 A1
- EP 2022062896 W
- WO 2021063813 A1


### Non-patent literature cited in the description


- **D. BENDIX**. *Polymer Degradation and Stability*, 03 January 1998, vol. 59 (1-3), 129-135
- **MANAVITEHRANI, IMAN et al.** Biomedical Applications of Biodegradable Polyesters.. *Polymers*, vol. 8, 1-20